# EUROPÄISCHE PATENTSCHRIFT

(11) **EP 1 787 671 B1**
(45) Veröffentlichungstag und Bekanntmachung des Hinweises auf die Patenterteilung: **19.12.2012**
(21) Anmeldenummer: 06021791.6
(22) Anmeldetag: 18.10.2006
(51) Int. Cl.: A61M 16/04

(54) **Halterung für Sprechventile und/oder Wärme-Feuchtigkeitstauscher**
Holder for speech valve and/or heat-moisture exchanger
Support pour valvule phonique et/ou échangeur thermo-hydrique

(30) Priorität: 16.11.2005 DE 102005055331
(43) Veröffentlichungstag der Anmeldung: 23.05.2007
(73) Patentinhaber: ISKIA GmbH & Co. KG, 38829 Harsleben (DE)
(72) Erfinder: Neubauer, Norbert, 38820 Halberstadt (DE)
(74) Vertreter: Pfeiffer, Rolf-Gerd

(56) Entgegenhaltungen:
- WO-A-94/01158
- DE-C1- 10 007 623
- DE-U1-202005 008 359

## Beschreibung

Die Erfindung betrifft eine Halterung für Sprechventile und/oder Wärme-Feuchtigkeitstauscher, die bei Tracheostomapflastern Anwendung findet.

Aus US 4,325,366 ist ein Sprechventil bekannt, das vermittels eines doppelseitig klebenden Pflasters um das Stoma eines Patienten befestigt wird, wobei das gesamte Ventil in eine trommelförmig Aufnahme eingesteckt wird. Dabei ist das Ventil einzig durch Klemmreibung in einer trommelförmigen Aufnahme lösbar fixiert. Eine vergleichbare Aufnahme wird von der Fa. Heimomed Heinze GmbH & Co. KG unter der Bezeichnung PRIM-AIR-STRIP vertrieben, wobei dort ebenfalls austauschbare Sprechventile und/oder Wärme-Feuchtigkeitstauscher in eine ebenfalls trommelförmige elastische Aufnahme nahezu vollständig eingedrückt werden. Eine zusätzliche Nut, die in ein entsprechendes Gegenstück des das Ventil oder den Wärme-Feuchtigkeitstauscher tragenden Gehäuses eingreift, sorgt hier für eine zusätzliche Sicherung genannter Baugruppen gegen unbeabsichtigtes Lösen und dadurch für einen festeren Sitz.

Eine vergleichbare, aber praktisch doppelt soweit vom Stoma abragende Vorrichtung für einen ebenfalls vermittels eines doppelseitigen Klebebandes angebrachten Wärme-Feuchtigkeitstauscher ist in EP 0387220 B2 beschrieben. Dort wird ein Wärme-Feuchtigkeitstauscher auf den oberen Rand einer trichterförmig ausgebildeten und auf das Stoma aufgeklebten Haltevorrichtung mittels Krallen eingeschnappt. Diese Vorrichtung weist den Nachteil einer relativ hohen, vom Stoma beabstandeten Bauform auf und genügt damit nicht ästhetischen Gesichtspunkten des Trägers.

In WO 99/29268 ist schließlich eine Sprechventilanordnung beschrieben, bei der ein Gehäuse zur Aufnahme eines Wärme-Feuchtigkeitstauscher und eines Ventils vorgesehen ist. Dieses Gehäuse wird ebenfalls mit einer Haltevorrichtung, die in bekannter Weise auf das Stoma aufgeklebt ist verbunden, wobei hier, im Gegensatz zu den vorstehend beschriebenen Vorrichtungen, das gesamte, das Sprechventil und einen Wärme-Feuchtetauscher aufnehmende Gehäuse nur am unteren Rand dieses Gehäuses über eine zirkular das proximale Gehäuseende umlaufenden Rippe, die in eine nutförmige, am Klebeband befestigte Halterung eingreift gehaltert ist. Bei dieser Art der Halterung kann es relativ leicht zum unbeabsichtigten Lösen des Ventilgehäuses kommen.

Allen vorstehenden Vorrichtungen ist gemein, dass sie keinen sicheren Sitz des Sprechventils und/oder Wärme-Feuchtigkeitstauschers gewährleisten, da bspw. beim Husten oder Niesen des Patienten erhebliche Drucke auftreten, die leicht Orkanstärke erreichen können, wodurch die Ventile regelrecht fortgeschleudert werden. Bis auf letztgenanntes Sprechventil weisen die bekannten Bauformen zusätzlich den Nachteil auf, dass sie sich beim Wechsel, bspw. zu Reinigungszwecken oder Austausch des Wärme-Feuchtigkeitstauschers, für den Patienten relativ schwer entnehmen lassen, weil der Hauptteil des Gehäuses von der Halterung umfangsmäßig erfasst ist.

Außerdem sind aus einem etwas entfernter liegenden Stand der Technik, wie bspw. in DE 100 07 623 C1, WO 94/01158 A1 und DE 20 2005 008 359 U1 beschrieben, Lösungen zur Befestigung von Baugruppen, wie Sprechventilen und/oder Wärme-Feuchtigkeitstauschem, bekannt, wobei sich diese jedoch ausschließlich auf deren Befestigung an Trachealkanülen beziehen. Da bei solchen Verbindungen aber relativ starre Partner (Trachealkanüle und die dort anzubringende Baugruppe) miteinander verbunden werden, spielen dort Dichtigkeitsprobleme, im Gegensatz zu vorliegender Erfindung, praktisch keine Rolle.

Der Erfindung liegt daher die Aufgabe zugrunde, eine Halterung für Sprechventile und/oder Wärme-Feuchtigkeitstauscher für Laryngetomierte oder Tracheotomierte anzugeben, die zum einen einen äußerst festen Sitz im eingebauten Zustand und eine flache Bauform und zum anderen eine problemlose Austauschbarkeit durch den Patienten gewährleistet.

Die Aufgabe wird durch die Merkmale des Patentanspruchs 1 gelöst. Vorteilhafte weitere Ausbildungen sind von den nachgeordneten Ansprüchen erfasst.

Die erfindungsgemäße Halterung zeichnet sich dadurch aus, das die Halterung für Sprechventile und/oder Wärme-Feuchtigkeitstauscher, in Form eines im wesentlichen zylindrischen Gehäuses, das das Sprechventil und/oder den Wärme-Feuchtigkeitstauscher trägt, respektive aufnimmt, wobei das Gehäuse am oder im Bereich seines proximalen Endes innwändig mit wenigstens einem Steg versehen ist, in den Haken, die im Bodenbereich eines Halterings vorgesehen sind, durch Verdrehung eingreifen, wobei zwischen den proximalen Anlageflächen des Gehäuses und kongruent zu diesen vorgesehenen Auflageflächen des Halterings im gegeneinander verspannten Zustand von Gehäuse und Haltering Dichtflächen gebildet sind.

Gegenüber bekannten Halterungen für den gleichen Zweck weist die erfindungsgemäße Lösung eine ausgesprochen komfortable Handhabung für den Patienten auf. Dieser kann das vollständige Sprechventil und/oder den Wärme-Feuchtigkeitstauscher durch einfache leichte Drehung einsetzen und entfernen, was für Reinigungszwecke oder Filterwechsel mehrmals täglich geschehen muss, wohingegen im arretierten Zustand eine absolut sichere festsitzende Arretierung gewährleistet ist. Dadurch, dass fast die gesamte Gehäusewandung des Sprechventils und/oder den Wärme-Feuchtigkeitstauschers mit den Fingern erfassbar ist, kann das Gehäuse vom Träger dieses Bauteils leicht zugänglich mit zwei Fingern erfasst werden.

Die Erfindung soll nachstehend anhand von Ausführungsbeispielen und schematischer Zeichnungen näher erläutert werden. Es zeigen:
- Fig. 1: eine erste Ausbildungsmöglichkeit der Halterung in einer seitlich tlw. geschnittenen Ansicht,
- Fig. 2: eine Ansicht auf den Bodenbereich einer Halterung nach Fig. 1,
- Fig. 3: einen vergrößerten ersten Teilschnitt Z aus Fig. 1 und
- Fig. 4: einen vergrößerten zweiten Teilschnitt Y aus Fig. 1.

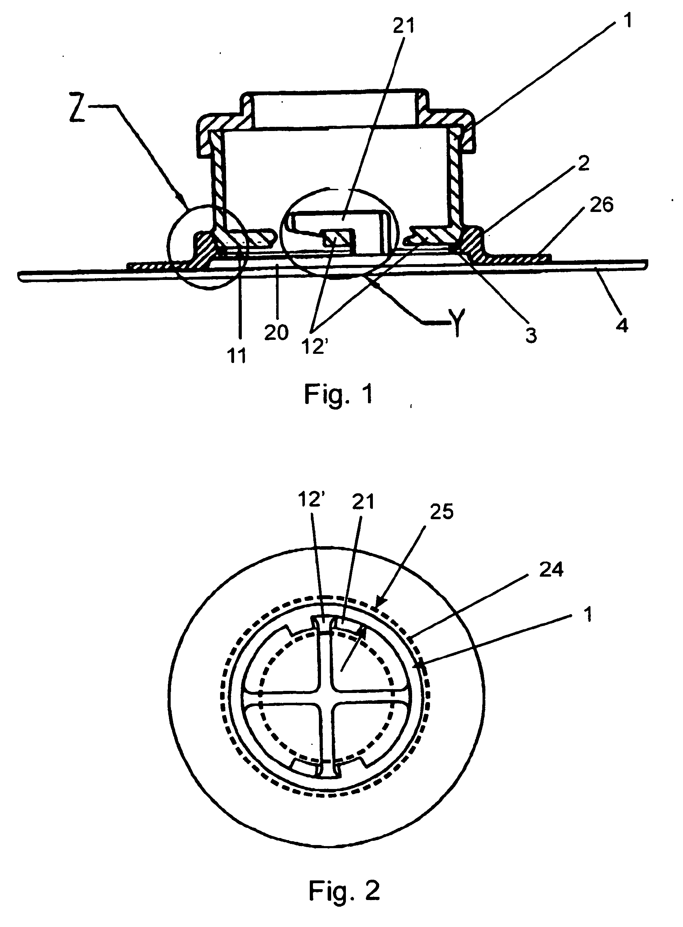

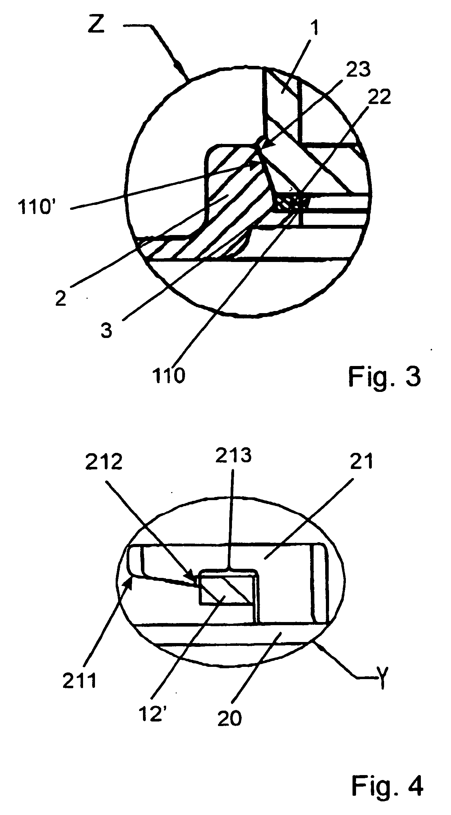

In Figur 1 ist schematisch eine erste Ausbildungsmöglichkeit der erfindungsgemäßen Halterung in einer seitlich teilweise geschnittenen Ansicht dargestellt. Dort ist zunächst ein im Wesentlichen zylindrisches Gehäuse 1 vorgesehen, in das ein Sprechventil und/oder den Wärme-Feuchtigkeitstauscher einsetzbar sind. Das Sprechventil befände sich in der nicht näher bezeichneten oberen Gehäusekappe und der Wärme-Feuchtigkeitstauscher würde vollständig in das Gehäuse 1 eingesetzt. Da diese beiden Baugruppen durch die Erfindung aber keine weitere Ausgestaltung erfahren, wurde aus Gründen der Übersichtlichkeit auf deren nähere Darstellung verzichtet. Im Rahmen der Erfindung ist wesentlich, dass das Gehäuse 1 am oder im Bereich seines proximalen Endes 11 innwändig mit wenigstens einem Steg 12' [vgl. Fig. 2, bspw. im Falle eines Wärme-Feuchtigkeitstauscher] versehen ist. Zum Eingriff der Stege 12' sind Haken 21 vorgesehen, die im Bodenbereich 20 eines Halterings 2 vorgesehen sind. In den Figuren 1 und 2 ist dabei der jeweils arretierte Zustand dargestellt. In diesem Zustand bilden sich zwischen Gehäuse 1 und Haltering 2, je nach Ausbildung, luftdichte Dichtflächen. Ist die Wandstärke des Gehäuses 1 hinreichend stark und das Gehäuse im Eingriffsbereich des Halterings rein zylindrisch ausgebildet, wirken als Dichtflächen die proximale Anlagefläche 110 des Gehäuses 1 und die kongruent zu dieser vorgesehene Auflagefläche 22 des Halterings 2 im gegeneinander verspannten Zustand von Gehäuse 1 und Haltering 2 (vgl. Fig. 3). Vorteilhaft ist dem Gehäuse 1 im proximalen Endbereich jedoch eine konische Fase derart gegeben, dass sich parallel zueinander verlaufende konische Dichtflächen zwischen der proximalen Anlagefläche 110' des Gehäuses 1 und der kongruent zu dieser vorgesehenen Auflagefläche 23 des Halterings 2 im gegeneinander verspannten Zustand von Gehäuse 1 und Haltering 2 ergeben. Durch die Spannkraft der erfindungsgemäß vorgesehenen Haken wird dann das Gehäuse 1 in den Haltering 2 hineingezogen und luftdicht abgedichtet.

Vorstehend beschrieben Sachverhalte bzgl. der Dichtflächen sind aus Fig. 3, die einen vergrößerten ersten Teilschnitt Z aus Fig. 1 darstellt, deutlich ersichtlich. Zum Ausgleich von Fertigungstoleranzen und/oder zur Erhöhung der Luftdichtheit der zu verbindenden Komponenten ist im Rahmen der Erfindung in weiterer Ausgestaltung vorgesehen, im Bereich der Auflageflächen 22 und/oder 23 des Halterings 2 zusätzlich einen elastischen Dichtring 3 einzusetzen.
Dazu ist es erforderlich, dass die vorgesehenen Haken 21 von dem benachbarten Wandungsbereich 24 des Halterings 2 nach innen soweit beabstandet angeordnet sind, dass vom verbleibenden Zwischenraum 25 die Wandung des Gehäuses 1 aufnehmbar ist. Dies wird in Fig. 2 verdeutlicht, die im Wesentlichen eine Ansicht auf den Bodenbereich (Fig. 1 von unten betrachtet) einer Ausführung nach Fig. 1 darstellen soll. Dabei soll der äußere strichlinierte Kreis den äußeren Innenumfang des Halterings 2 darstellen und der innere strichlinierte Kreis die lichte Durchlassöffnung des Halterings 2 in Richtung Stoma. Zwischen beiden genannten Kreisen befindet sich der Bodenbereich des Halterings 2, auf dem das Gehäuse 1 derart aufsitzt, dass sich die Gehäusewandung zwischen dem äußeren strichlinierten Kreis und den auf dem Bodenbereich vorgesehenen Haken 21 befindet.

Figur 4 zeigt einen vergrößerten zweiten Teilschnitt Y aus Fig. 1, der sich mit der besonderen Ausgestaltung der erfindungsgemäß vorgesehenen Haken 21 befasst. Dort ist im Querschnitt ein Abschnitt eines Stegs 12' im bereits in den Haken 21 eingerasteten Zustand dargestellt. Im nicht dargestellten unverspannten Zustand der Vorrichtung wird dieser Steg 12', von links kommend unter den Haken 21 eingedreht. Um das Einlaufen des Stegs 12' zu erleichtern, ist den Haken 21 dazu ein verrundeter Einlaufbereich 211 gegeben. Um den Stegen einen gesicherten Sitz im verspannten Zustand zu verleihen, sind die Haken 21 vorteilhaft mit einem Rastgesperre 212 versehen, denen ein Aufnahmebereich 213 für die Nase 12 oder den Steg 12' nachgeordnet ist.

Im Rahmen der Erfindung sind das Gehäuse 1, der Haltering 2 und die Haken 21 aus einem steifen und wenig elastischen Material, wie Polypropylen, Polycarbonat, SAN oder PS gefertigt, wohingegen der elastische Dichtring 3 bevorzugt aus Silikon gefertigt ist. Andere bei medizinischen Anwendungen für den vorgesehenen Zweck übliche Materialien liegen selbstverständlich ebenfalls im Rahmen der Erfindung.

Verschiedene Arten der Anordnung eines vorgesehenen Dichtrings 3 sind Gegenstand der Ansprüche 8, 9 und 10.

Gleichzeitig ist in Fig. 1 die Anbringung des Halterings 2 auf einem Pflaster 4 angedeutet. Zu diesem Zweck ist der Haltering 2 peripher so ausgebildet, dass der Haltering 2 in einen Flansch 26 ausläuft, der mit dem Pflaster 4, welches über dem nicht dargestellten Stoma abgeklebt wird, bspw. verschweißt ist. Dabei ist der Flansch 26 so dünn ausgeführt ist, dass er eine hinreichende Elastizität aufweist.

Im Rahmen der Erfindung ist der Haltering 2 mit all seinen Bestandteilen, insbesondere mit den Haken 21, besonders vorteilhaft durch ein einstückiges Spritzteil gebildet.

Da es bzgl. des Halterings 2 und der Art der Befestigung des Gehäuses 1 keine Beschränkungen hinsichtlich des Einsatzes dieser Halterung gibt, sei vorsorglich erwähnt, dass der Haltering 2 selbstverständlich auch den distalen Aufnahmebereich einer Kanüle 5, egal ob Außen- oder Innenkanüle, bilden kann.

In weiterer Ausgestaltung der Erfindung sei erwähnt, ohne dass es in beiliegenden Figuren detailliert dargestellt ist, dass den Stegen 12' in Richtung des Aufnahmebereichs 213 der Haken 21 ein verrundeter Querschnitt oder zumindest in Richtung des Rastgesperres 212 eine Fase gegeben ist. Eine derartige Fase kann am Beispiel von Stegen (vgl. Fig. 4) bspw. links oben am Steg angebracht sein, wodurch ein leichteres Lösen des Gehäuses durch entgegengesetzte Verdrehung befördert wird.

Bereits bekannte Wärme-Feuchtigkeitstauscher, die in zylindrischen Gehäusen untergebracht sind, weisen fast durchgängig Stege in Form eines Bodenkreuzes (wie aus Fig. 2 ersichtlich) auf, welches einzig dazu vorgesehen ist, den Wärme-Feuchtigkeitstauscher am Hineinrutschen in das Stoma zu hindern. Vorteilhaft lassen sich diese Kreuze für die hier vorgesehene Art der Gehäusebefestigung nutzen, ohne dass es besonderer weiterer Umgestaltungen dieser Einsätze bedarf. Bevorzugt, aber die Erfindung nicht darauf beschränkend, werden in diesen Fällen zwei einander gegenüberliegende Haken 21 vorgesehen. Bei dünneren und damit elastischeren Gehäusewandungen können im Beispiel auch vier sich gegenüberstehende und jeweils um 90° versetzte Haken 21 vorgesehen sein, was jedoch den einfachen Einsatz und Austausch des Gehäuses durch den Patienten etwas erschwert.

Wie oben bereits erwähnt, weisen bekannte Sprechventile, die durch ein zylinderförmiges Gehäuse getragen werden, solche Bodenkreuze nicht auf. In diesen Fällen bedarf es einer Umgestaltung dieser Gehäuse, was sich ebenfalls leicht in Form eines einstückig gespritzten Gehäuses realisieren lässt. Alle übrigen beschriebenen Merkmale der Erfindung bleiben dabei unverändert.

### Bezugszeichenliste

- 1 -: Gehäuse
- 11 -: proximales Gehäuseende
- 110, 110' -: proximale Anlageflächen
- 12 -: Nase
- 12' -: Steg
- 2 -: Haltering
- 21 -: Haken
- 211 -: verrundeter Einlaufbereich
- 212 -: Rastgesperre
- 213 -: Aufnahmebereich für Nase oder Steg
- 22, 23 -: Auflageflächen
- 24 -: dem Haken benachbarter Wandungsbereich des Halterings
- 25 -: Zwischenraum
- 26 -: Flansch
- 3 -: Dichtring
- 4 -: Pflaster
- 5 -: Kanüle
- Y, Z -: Teilschnitte

## Patentansprüche

1. Halterung für Sprechventile und/oder Wärme-Feuchtigkeitstauscher, die auf einem Tracheostomapflaster angeordnet ist, beinhaltend ein im wesentlichen zylindrisches Gehäuse (1), das das Sprechventil und/oder den Wärme-Feuchtigkeitstauscher trägt, respektive aufnimmt, **dadurch gekennzeichnet, dass** das Gehäuse (1) am oder im Bereich seines proximalen Endes (11) innwändig mit wenigstens einem Steg (12') versehen ist, in den Haken (21), die im Bodenbereich (20) eines Halterings (2) vorgesehen sind, durch Verdrehung eingreifen, wobei zwischen den proximalen Anlageflächen (110 und/oder 110') des Gehäuses (1) und kongruent zu diesen vorgesehenen Auflageflächen (22; 23) des Halterings (2) im gegeneinander verspannten Zustand von Gehäuse (1) und Haltering (2) Dichtflächen gebildet sind, wobei das Gehäuse (1) und der Haltering (2), sowie die Haken (21) aus einem steifen und wenig elastischen Material gefertigt sind und der Haltering (2) mit einem Flansch (26), der mit dem Tracheostomapflaster (4) verscheißt ist, versehen ist, wobei der Flansch (26) seinerseits so dünn ausgeführt ist, dass er wieder elastisch ist.

2. Halterung nach Anspruch 1, **dadurch gekennzeichnet**, das im Bereich der Auflageflächen (22; 23) des Halterings (2) zusätzlich ein elastischer Dichtring (3) vorgesehen ist.

3. Halterung nach Anspruch 1, **dadurch gekennzeichnet, dass** die Haken (21) von dem benachbarten Wandungsbereich (24) des Halterings (2) nach innen soweit beabstandet angeordnet sind, dass vom verbleibenden Zwischenraum (25) die Wandung des Gehäuses (1) aufnehmbar ist.

4. Halterung nach Anspruch 1, **dadurch gekennzeichnet, dass** den Haken (21) ein verrundeter Einlaufbereich (211) gegeben ist.

5. Halterung nach Anspruch 1, **dadurch gekennzeichnet, dass** die Haken (21) mit einem Rastgesperre (212) versehen sind, denen ein Aufnahmebereich (213) für den Steg (12') nachgeordnet ist.

6. Halterung nach Anspruch 1, **dadurch gekennzeichnet, dass** das Gehäuse (1) und der Haltering (2), sowie die Haken (21) aus einem Material, wie Polypropylen, Polycarbonat, SAN oder PS gefertigt sind.

7. Halterung nach Anspruch 2, **dadurch gekennzeichnet, dass** der elastische Dichtring (3) bevorzugt aus Silikon gefertigt ist.

8. Halterung nach Anspruch 2 oder 7, **dadurch gekennzeichnet, dass** der elastische Dichtring (3) ausschließlich plan über den Auflageflächen (22) im Bodenbereich (20) des Halterings (2) und korrespondierend zur proximalen Anlagefläche (110) des Gehäuses (1) erstreckend angeordnet ist.

9. Halterung nach Anspruch 2 oder 7, **dadurch gekennzeichnet, dass** der elastische Dichtring (3) ausschließlich über den konischen Innenrandbereich der Auflagerflächen (23) des Halterings (2) und korrespondierend zur proximalen Anlagefläche (110') des Gehäuses (1) erstreckend angeordnet ist.

10. Halterung nach Anspruch 2 oder 7, **dadurch gekennzeichnet, dass** der elastische Dichtring (3) sowohl über den Auflageflächen (22) des Bodenbereichs (**20**) als auch über den konischen Innenrandbereich der Auflageflächen (23) des Halterings (2) und korrespondierend zu den proximalen Anlageflächen (110 und 110') des Gehäuses (1) erstreckend angeordnet ist oder im gespannten Zustand in genannte Flächen des Halterings (2) eingepresst ist.

11. Halterung nach Anspruch 6, **dadurch gekennzeichnet, dass** der Haken (21) so dünn ausgeführt ist, dass er wieder elastisch ist.

12. Halterung nach Anspruch 1, **dadurch gekennzeichnet, dass** der Haltering (2) und die Haken (21) durch ein einstückiges Spritzteil gebildet sind.

13. Halterung nach Anspruch 1 oder 5, **dadurch gekennzeichnet, dass** den Stegen (12') in Richtung des Aufnahmebereichs (213) der Haken (21) ein verrundeter Querschnitt oder zumindest in Richtung des Rastgesperres (212) eine Fase gegeben ist.

## Claims

1. A mount for phonation valves and/or Heat and Moisture Exchangers which is arranged upon a tracheostoma plaster, comprising a substantially cylindrical housing (1), which supports and receives, respectively, the phonation valve and/or the heat and moisture exchanger, **characterized in that** the housing (1) at its proximal end (11) or within the range of the proximal end (11) is internally provided with at least one web (12') into which hooks (21), provided in the bottom range (20) of a retaining ring (2) engage by rotation, wherein sealing faces are formed between the proximal bearing faces (110 and/or 110') of the housing (1) and congruently provided to the latter mounting surfaces (22, 23) of the retaining ring (2) when the housing (1) and the retaining ring (2) are mutually in a tensioned state, wherein the housing (1) and the retaining ring (2), as well as the hooks (21) are made of a rigid and poorly elastic material, and wherein the retaining ring (2) is provided with a flange (26) which is welded to the tracheostoma plaster (4), wherein the flange (26) in turn is designed such thin that it again is elastic.

2. A mount as claimed in claim 1, **characterized in that** an elastic sealing ring (3) is additionally provided in the range of the mounting surfaces (22, 23) of the retaining ring (2).

3. A mount as claimed in claim 1, **characterized in that** the hooks (21) are arranged spaced to the adjacent wall range (24) of the retaining ring (2) inwardly so that the wall of the housing (1) may receive the remaining interspaces (25).

4. A mount as claimed in claim 1, **characterized in that** the hook (21) is provided with a rounded insertion range (211).

5. A mount as claimed in claim 1, **characterized in that** the hooks (21) are provided with an arresting mechanism (212) followed by a reception range (213) for the web (12').

6. A mount as claimed in claim 1, **characterized in that** the housing (1) and the retaining ring (2) as well as the hooks (21) are made of a material such as polypropylene, polycarbonate, SAN or PS.

7. A mount as claimed in claim 2, **characterized in that** the elastic seal ring (3) is preferably made of silicone.

8. A mount as claimed in claims 2 or 7, **characterized in that** the elastic seal ring (3) is exclusively arranged in plane extension over the mounting face (22) in the bottom range (20) of the retaining ring (2) and in corresponding relation to the proximal bearing face (110) of the housing (1).

9. A mount as claimed in claims 2 or 7, **characterized in that** the elastic seal ring (3) is exclusively arranged extending on the conical inner rim area of the mounting face (23) of the retaining ring (2) in corresponding relation to the proximal bearing face (110') of the housing (1).

10. A mount as claimed in claims 2 or 7, **characterized in that** the elastic seal ring (3) is exclusively arranged extending over mounting faces (22) of the bottom range (20) as well as over the conical inner rim area of the mounting face (23) of the retaining ring (2) and corresponding to the proximal bearing faces (110 and 110') of the housing (1), or when in a tensioned state is pressed into said faces of the retaining ring (2).

11. A mount as claimed in claim 6, **characterized in that** the hook (21) is designed so thin that it turns elastic again.

12. A mount as claimed in claim 1, **characterized in that** the retaining ring (2) and the hooks (21) are formed in one piece by injection moulding.

13. A mount as claimed in claim 1 or 5, **characterized in that** the webs (12') in direction of the reception area (213) for the hooks (21) are provided with a rounded cross-section or at least in direction of the arresting mechanism (212) with a chamfer.

## Revendications

1. Le dispositif destiné à assurer la fixation de valves permettant au patient de parler, et/ou d'un échangeur de chaleur et d'humidité, disposés sur un pansement adhésif de trachéostomie, se compose d'un boîtier essentiellement cylindrique (1) renfermant la valve permettant de parler et/ou l'échangeur de chaleur et d'humidité, est **caractérisé en ce que** le boîtier (1) est doté sur la paroi intérieure de son extrémité proximale (11) ou à proximité de celle-ci, d'au moins un pont (12') sur lequel s'attachent par torsion des crochets (21) prévus au fond (20) d'une bague de maintien (2), ce qui entraîne la formation de surfaces de fermeture étanches entre les faces proximales (110 et/ou 110') du boîtier (1) et en position congruente par rapport à celles-ci, les surfaces d'appui (22;23) de la bague de maintien (2), le boîtier et (1) et la bague de maintien (2) se trouvant dans un état de tension l'un par rapport à l'autre; le boîtier (1) et la bague de maintien (2) ainsi que les crochets (21) étant fabriqués dans un matériau rigide et peu élastique et la bague de maintien (2) étant dotée d'une bride (26) soudée au pansement de trachéostomie (4), qui, de son côté est si mince qu'elle en redevient élastique.

2. Le dispositif de fixation selon la revendication 1 est **caractérisé en ce que** se trouve en plus une bague d'étanchéité élastique (3) dans la zone des surfaces d'appui (22; 23) de la bague de maintien (2).

3. Le dispositif de fixation selon la revendication 1 est **caractérisé en ce que** la distance qui sépare les crochets (21) et la paroi voisine (24) de la bague de maintien (2) est suffisante pour placer dans cet interstice (25) la paroi du boîtier (1).

4. Le dispositif de fixation selon la revendication 1 est **caractérisé en ce que** les crochets (21) disposent d'une zone d'entrée arrondie (211).

5. Le dispositif de fixation selon la revendication 1 est **caractérisé en ce que** les crochets (21) sont dotés d'un dispositif d'encliquetage (212) suivi d'une zone de réception (213) pour le pont (12').

6. Le dispositif de fixation selon la revendication 1 est **caractérisé en ce que** le boîtier (1) et la bague de maintien (2) ainsi que les crochets (21) sont fabriqués à partir de matériaux tels que polypropylène, polycarbonate, SAN ou PS.

7. Le dispositif de fixation selon la revendication 2 est **caractérisé en ce que** la bague d'étanchéité élastique (3) est fabriquée de préférence à partir de silicone.

8. Le dispositif de fixation selon 2 ou 7 est **caractérisé en ce que** la bague d'étanchéité (3) doit être disposée de manière plane au-dessus des surfaces d'appui (22) situées au fond de la bague de maintien (2) et de manière allongée par rapport à la face de contact proximale (110) du boîtier (1).

9. Le dispositif de fixation selon les revendications 2 ou 7 est **caractérisé en ce que** la bague d'étanchéité (3) doit être disposée uniquement au-dessus de la zone du bord intérieur des surfaces d'appui (232) situées au fond de la bague de maintien (2) et de manière allongée par rapport à la surface d'appui proximale (110') du boîtier (1).

10. Le dispositif de fixation selon les revendications 2 ou 7 est **caractérisé en ce que** la bague d'étanchéité élastique (3) est disposée aussi bien au-dessus des surfaces d'appui (22) de la zone du fond (20) qu'au-dessus de la zone du bord intérieur conique des surfaces d'appui (23) de la bague de maintien (2) et ce soit de manière allongée par rapport aux faces de contact (110 et 110') du boîtier (1), soit de manière comprimée en état de tension dans les surfaces mentionnées de la bague de maintien (2).

11. Le dispositif de fixation selon la revendication 6 est **caractérisé en ce que** le crochet (21) est si mince qu'il en devient élastique.

12. Le dispositif de fixation selon la revendication 1 est **caractérisé en ce que** la bague de maintien (2) et les crochets (21) forment une seule pièce moulée par injection.

13. Le dispositif de fixation selon les revendications 1 et 5 est **caractérisé en ce que** les ponts (12') orientés en direction de la surface d'appui (213) des crochets (21) présentent une section arrondie ou au moins un chanfrein orienté vers le dispositif d'encliquetage (212).
